Europäisches Patentamt

European Patent Office

Office européen des brevets

(19)

(11) Publication number: **0 144 434**
**A1**

## EUROPEAN PATENT APPLICATION

published in accordance with Art. 158(3) EPC

(21) Application number: 84901610.0

(22) Date of filing: 18.04.84

Data of the international application taken as a basis:

(86) International application number:
PCT/JP84/00196

(87) International publication number:
WO84/04244 (08.11.84 84/26)

(51) Int. Cl.⁴: **A 61 K 9/10**
A 61 K 31/195, A 61 K 31/23

(30) Priority: 20.04.83 JP 69712/83

(43) Date of publication of application:
19.06.85 Bulletin 85/25

(84) Designated Contracting States:
AT BE CH DE FR GB LI LU NL SE

(71) Applicant: AMERICAN HOSPITAL SUPPLY
CORPORATION
One American Plaza
Evanston, IL 60201(US)

(72) Inventor: OHASHI, Hiroyuki
958, Kashimada Saiwai-ku
Kawasaki-shi Kanagawa-ken 211(JP)

(72) Inventor: HATTORI, Toshiaki
683-8, Ozenji Aso-ku
Kawasaki-shi Kanagawa-ken 215(JP)

(72) Inventor: KOBAYASHI, Tetsuo
283-6, Nasecho Totsuka-ku
Yokohama-shi Kanagawa-ken 245(JP)

(72) Inventor: HASHIMOTO, Shigebumi
3726-2, Akuwacho Seya-ku
Yokohama-shi Kanagawa-ken 246(JP)

(72) Inventor: TAKAMI, Toru
20-14, Kurihamadai 2-chome
Yokosuka-shi Kanagawa-ken 239(JP)

(74) Representative: Vossius Vossius Tauchner Heunemann
Rauh
Siebertstrasse 4 P.O. Box 86 07 67
D-8000 München 86(DE)

(54) AMINO ACID-CONTAINING FAT EMULSION.

(57) An intravenously administrable, amino acid-containing fat emulsion having an emulsion stability improved with at least one compound selected from the group consisting of N-acyllysine, citrulline, N-acylarginine, and N-acylornithine.

EP 0 144 434 A1

## Specification

## An amino acid-containing fat emulsion

Field of the Invention

This invention relates to a novel fat emulsion containing amino acids, which is stable and suitable for intravenous injection.

Background of the Invention

Remarkable progress has recently been made in an intravenous hyperalimentation (IVH) as a means for surgical nutrition, and IVH has been contributing greatly to saving the lives of patients. There are known fat, sugar and amino acid parenteral solutions for nourishment. These solutions are conveniently combined with each other to use depending upon energy or nitrogen required by patients. If IVH solution consists mainly of a sugar solution, there is no alternative but to inject it through the central veins, since it is a hypertonic solution. Its control is, therefore, difficult, as there is every likelihood that complications may occur. On the other hand, an IVH solution containing a fat emulsion as a main source of energy has the advantage of being suitable for injection through the peripheral veins, but it has a drawback which has failed to be overcome for a long time. Namely, its pharmaceutical stability is lost quickly if it is mixed with an amino acid, especially, lysine, arginine or salts thereof, electrolyte, or the like.

- 1 -

An amino acid parenteral solution is inefficient for use in a living body, since an amino acid, if used alone, is likely to be consumed as a source of energy rather than as a material for protein synthesis in the event the body has a shortage of energy as a result of, for example, surgical stress. Therefore, it is advisable that an amino acid parenteral solution be used with another appropriate source of energy.

Fat is desirable as such energy source. However, when fat is added to a parenteral solution containing an amino acid, especially lysine, arginine or salts thereof, the resulting amino acid-containing fat emulsion is unstable.

Disclosure of the Invention

The inventors of this invention have been eagerly engaged in a research program to develop an amino acid-containing fat emulsion which remains pharmaceutically stable for a long time, lends itself to high calorie administration through the peripheral veins, and promotes protein anabolism. It is desirable from the nutritional point of view that lysine and arginine, which are nutritionally important, are contained in an amino acid-containing fat emulsion. However, if lysine, arginine or a salt thereof is added to a fat emulsion or an amino acid-containing fat emulsion, the stability of the resulting emulsion is greatly reduced as described above. The inventors

- 2 -

have studied to improve such disadvantage and have found that if lysine or arginine in the form of N-acyl derivative is added to fat emulsion, the resulting emulsion remains stable and that N-acyl-ornithine or citrulline may be used as a substitute for N-acyl-arginine. As a result, they have achieved this invention.

This invention provides a fat emulsion containing an amino acid, characterized in that the fat emulsion contains at least one member selected from a group consisting of N-acyl-lysine, citrulline, N-acyl-arginine and N-acyl-ornithine.

It is desirable from the nutritional point of view that the amino acid-containing fat emulsion of the present invention contains at least one, preferably all members of essential amino acids: L-isoleucine, L-leucine, L-valine, L-methionine, L-threonine, L-phenylalanine, and L-tryptophan, in addition to at least one member selected from the group consisting of N-acyl-L-lysine, N-acyl-L-arginine, N-acyl-L-ornithine and L-citrulline. The fat emulsion of the present invention may contain non-essential amino acids, such as L-histidine, L-serine, L-proline, L-alanine, glycine, L-tyrosine or L-cysteine (in the form of a free amino acid, hydrochloride, hydrate or the like). These essential and non-essential amino acids may be contained in the emulsion in the form of a free acid and any derivatives which can be metabolized in the living body like a free amino acid, such as salts, esters, or N-acyl-derivatives.

In the fat emulsion of the present invention, it is most preferred that all lysine is present in the form of N-acyl-derivative. However, lysine may be present to some extent in the form of other than N-acyl form, for example in the form of an organic or inorganic acid salt, or a free acid. Thus, it is possible to use the N-acyl form of lysine exclusively, or a mixture of the N-acyl and free forms. Alternatively, it is possible to use a mixture of lysine in N-acyl form and in the form of an organic acid salt, or a mixture which further contain free lysine. The emulsion may contain, say, $4 \times 10^{-3}$ to $5 \times 10^{-4}$ % (mole/V) of lysine in N-acyl form.

If any other form of lysine is used, it may be used in the quantity of, say, $2.5 \times 10^{-4}$ to $5 \times 10^{-4}$ % (mole/V).

The urea cycle amino acid such as citrulline, N-acyl-arginine, N-acyl-ornithine may be used in the quantity of, say, $4 \times 10^{-3}$ to $5 \times 10^{-4}$% (mole/V). If a urea cycle amino acid other than citrulline, N-acyl-arginine and N-acyl-ornithine, for example, arginine or ornithine or salts thereof is used, it is suitable to use it in the quantity of, say, $2.5 \times 10^{-4}$ to $5 \times 10^{-4}$ % (mole/V).

Amino acids contained in the fat emulsion of the present invention can be employed in the N-acyl form. Such acyl groups include various physiological acid residues, such as:

(1) Residue of a monovalent saturated fatty acid, such as acetic acid, propionic acid, caproic acid, palmitic acid or stearic acid; a divalent saturated fatty acid, such as malonic acid or succinic acid; a monovalent unsaturated fatty acid, such as oleic acid, linoleic acid or linolenic acid; a divalent unsaturated fatty acid, such as fumaric acid or maleic acid;

(2) residue of an aliphatic oxyacid, such as lactic acid or malic acid;

(3) residue of an aliphatic keto-acid, such as pyruvic acid or acetoacetic acid; and

(4) other formyl groups.

Acylation may be effected selectively in the α and ε positions of L-lysine, the α and δ positions of L-ornithine or the α position of L-arginine.

N-acyl-L-lysine, N-acyl-L-arginine and N-acyl-L-ornithine may usually be used in the quantity of 0.1 to 0.5 W/V %. L-citrulline may be used as a substitute for L-arginine in the quantity of usually 0.1 to 0.5 W/V %.

The upper concentration limit of the amino acids in the fat emulsion may experimentally be determined within ranges not losing a proper degree of the emulsion stability. The lower limit of the amino acids is the quantity which is nutritionally required. The fat emulsion of the present invention may have a total amino acid content of 1 to 15 W/V %, preferably 4 to 6 W/V %.

The fat which can be employed for this invention is, for example, fully refined cotton seed, sesame, peanut, olive, safflower or soy bean oil. Soy bean oil is particularly preferable, since it does not have any substantial side effect on the living body. The fat emulsion of the present invention may have a fat content of 1 to 15 W/V %, preferably 3 to 5 W/V %.

It is desirable to use as an emulsifying agent the yolk or soy bean lecithin which has been fully purified, and does not contain any pyrogenic substance. It is suitable to use it in the quantity of 10 to 50 W/V % of the fat.

The parenteral solution may further contain a thickening agent and a carbohydrate which is incorporated as a source of energy. As the carbohydrate, it is appropriate to use a sugaralcohol, such as sorbitol, xylitol or glycerol, which does not undergo any browning reaction with an amino acid, in the quantity of 3 to 15 W/V %.

The fat emulsion of this invention can be prepared, for example, by dispersing a fat and an emulsifying agent in distilled water for injection, emulsifying the dispersion under pressure, adding an amino acid and a carbohydrate thereinto simultaneously, and emulsifying the mixture under pressure again. It is also possible to prepare it by dispersing a fat, a carbohydrate and an emulsifying agent fully in distilled water for injection, emulsifying the mixture under pressure, adding an amino acid, and emulsifying it under pressure again.

The fat emulsion of this invention may also contain, for example, a vitamin such as vitamin A, K, E, $D_2$, a pH controller such as acetic acid, malic acid NaOH, KOH or a lipid stabilizer such as α-tocopherol, if required.

An attempt was made to confirm that the addition of N-acyl-lysine, N-acyl-arginine, N-acyl-ornithine or citrulline to a fat emulsion as hereinabove set forth would enable the preparation of a stable amino acid-containing fat emulsion. It was made by adding the amino acids to 5 W/V % of soy bean oil and 2 W/V % of yolk lecithin, emulsifying the mixture, and sterilizing the emulsion at 120°C for 20 minutes under pressure. The difference in relative particle diameter was measured before and after the sterilization. The results are shown in TABLES1 and 2.

TABLE 1

| Amino Acids* | M-VALUE | |
| | Before Sterilization | After Sterilization |
| --- | --- | --- |
| L-lysine acetate | 15 | 76 |
| L-lysine hydrochloride | 9 | 84 |
| α-N-acetyl-L-lysine | 6 | 14 |
| ε-N-acetyl-L-lysine | 7 | 60 |
| ε-N-propionyl-L-lysine | 16 | 22 |

* 2.23 millimoles

The m-Value, which is obtained from the dilution rate and the absorbancy of a solution at 500 nm, is proportional to the average particle diameter. From Table 1, it can be seen that the m-Value of the fat emulsion to which L-lysine acetate or L-lysine hydrochloride has been added becomes larger after sterilization. This demonstrates that particles of the fat emulsions agglomerated. On the other hand, it can be seen that the m-Value of the fat emulsion to which N-acyl derivative has been added is relatively smaller after sterilization, which demonstrates that the fat emulsions have excellent emulsion stability.

0144434

TABLE 2

| Amino Acids* | M-VALUE | |
| | Before Sterilization | After Sterilization |
| --- | --- | --- |
| L-arginine-L-aspartic acid salt | 18 | 113 |
| L-arginine-L-glutamic acid salt | 17 | 110 |
| L-arginine | 14 | 73 |
| L-ornithine acetate | 13 | 74 |
| L-citrulline | 7 | 33 |
| α-N-acetyl-L-arginine | 8 | 14 |
| α-N-acetyl-L-ornithine | 22 | 23 |
| δ-N-acetyl-L-ornithine | 12 | 21 |

* 2.45 millimoles

From Table 2, it can be seen that arginine, salts of ariginine and salts of ornithine harm the emulsion stability, while citrulline, N-acyl arginine and N-acyl ornithine do not harm the emulsion stability. According to this invention, it is possible to obtain, for example, an amino acid-containing fat emulsion which contains at least one member selected from the group consisting of N-acyl-L-lysine, L-citrulline, N-acyl-L-arginine and N-acyl-L-ornithine, and at least one fully refined vegetable oil as a fat,

- 9 -

and which is emulsified by lecithin obtained from egg yolk or soy beans.

The emulsion particles according to this invention remain stable and do not agglomerate to form oil droplets, despite sterilization at a high temperature and a high pressure, and a long period of storage.

Example

The present invention will be explained in the following EXAMPLES.

EXAMPLE 1

| | |
|---|---|
| L-threonine | 1.8 g |
| L-serine | 2.6 g |
| L-proline | 5.0 g |
| glycine | 6.3 g |
| L-alanine | 3.2 g |
| L-valine | 3.0 g |
| L-methionine | 2.4 g |
| L-isoleucine | 3.1 g |
| L-leucine | 4.1 g |
| L-tryptophan | 0.7 g |
| L-phenylalanine | 2.5 g |
| L-histidine | 1.3 g |
| ε-N-acetyl-L-lysine | 4.2 g |
| L-citrulline | 4.3 g |
| L-cysteine hydrochloride·1H$_2$O | 0.2 g |

- 10 -

| | |
|---|---|
| soybean oil | 50.0 g |
| yolk lecithin | 20.0 g |
| sorbitol | 30.0 g |
| glycerol | 30.0 g |
| retinol acetate | 600 μg |
| ergocalciferol | 2.5 μg |
| DL-α-tocopherol | 66.7 mg |
| phylloquinone | 100 μg |

The amino acids, sorbitol and glycerol were dissolved in 700 ml of distilled water for injection.

Vitamins and lecithin were added to, and dissolved in soy bean oil.

They were fully dispersed in a homomixer, and distilled water for injection was added to make a total volume of one liter. The dispersion was emulsified in a high pressure jet emulsifier at a pressure of 650 kg/cm$^2$ to yield an emulsion containing 5 W/V % of soy bean oil and 4.5 W/V % of amino acids. The emulsion was filtered by a cellulose membrane having a mesh size of 0.45 micron, and the filtrate was poured into a vial. It was sterilized at 121°C for 20 minutes to yield an amino acid-containing fat emulsion.

EXAMPLE 2

An amino acid-containing fat emulsion was obtained by repeating the procedures of EXAMPLE 1, except that 4.5 g of ε-N-propionyl-L-lysine was used instead of 4.2 g of ε-N-acetyl-L-lysine, and 5.3 g of α-N-acetyl-L-arginine instead of 4.3 g of L-citrulline.

COMPARATIVE EXAMPLE (A)

An amino acid-containing fat emulsion was obtained by repeating the procedures of EXAMPLE 1, except that 4.6 g of an acetate of L-lysine was used instead of 4.2 g of ε-N-acetyl-L-lysine.

COMPARATIVE EXAMPLES (B) AND (C)

The procedures of EXAMPLE 1 were repeated to prepare an amino acid-containing fat emulsion (B) except for the use of 4.6 g of L-lysine acetate instead of 4.2 g of ε-N-acetyl-L-lysine, and 4.3 g of L-arginine instead of 4.3 g of L-citrulline, and an amino acid-containing fat emulsion (C) except for the elimination of ε-N-acetyl-L-lysine and L-citrulline.

The m-values of the emulsions according to EXAMPLES 1 and 2 and COMPARATIVE EXAMPLES (A), (B) and (C) were measured before and after sterilization, and the appearance of each emulsion was inspected after storage at 40°C. The results are shown in

- 12 -

TABLE 3.

TABLE 3

| | M-VALUE | | Appearance after storage at 40°C |
|---|---|---|---|
| | Before sterilization | After sterilization | (weeks passed) |
| EXAMPLE 1 | 12 | 75 | Unchanged (18) |
| EXAMPLE 2 | 8 | 17 | Unchanged (18) |
| COMPARATIVE EXAMPLE (A) | 12 | 90 | Oil drops (5) |
| COMPARATIVE EXAMPLE (B) | 12 | 107 | Oil drops (1) |
| COMPARATIVE EXAMPLE (C) | 16 | 24 | Unchanged (18) |

It is noted that all of the emulsions obtained in EXAMPLES 1 and 2 constitute an improvement over that of COMPARATIVE EXAMPLES (A) and (B). The emulsion of COMPARATIVE EXAMPLE (C) shows an improved emulsion stability, because the emulsion neither contain ε-N-acetyl-L-lysine nor L-citrulline.

A commercially available amino acid-containing fat emulsion "NUTRIFUNDIN" (trade mark; product of Brawn, West Germany) was employed as COMPARATIVE EXAMPLE (D). It was presumed to be a product prepared in accordance with the disclosure of U.S. Patent No. 3,793,450. It and the emulsion of EXAMPLE 1 were stored at 40°C for 18 weeks, and compared thereafter in average particle diameter and particle size distribution by a centrifugal particle size distribution measuring device. The results are shown in TABLES 4 and 5.

TABLE 4

| | Average particle diameter (µm). | Cumulative distribution (%) | |
|---|---|---|---|
| | | 0.80 µm or above | 0.20 µm or above |
| EXAMPLE 1 | 0.08 | 0 | 11.4 |
| COMPARATIVE EXAMPLE (D) | 0.31 | 12.7 | 75.6 |

TABLE 5 shows the results which represent on a volume basis a histogram for the particle size distribution determined by centrifugal sedimentation.

TABLE 5

Particle size distribution

| diameter (µm) | DISTRIBUTION (%) | |
|---|---|---|
| | EXAMPLE 1 | COMPARATIVE EXAMPLE (D) |
| more than 2.00 | 0.0 | 0.0 |
| 2.00-1.88 | " | 0.1 |
| 1.88-1.76 | " | 1.1 |
| 1.76-1.64 | " | 1.0 |
| 1.64-1.52 | " | 0.1 |
| 1.52-1.40 | " | 1.2 |
| 1.40-1.28 | " | 1.3 |

| | | |
|---|---|---|
| 1.28-1.16 | " | 1.6 |
| 1.16-1.04 | " | 1.5 |
| 1.04-0.92 | " | 1.8 |
| 0.92-0.80 | " | 3.1 |
| 0.80-0.68 | " | 5.7 |
| 0.68-0.56 | 0.3 | 7.3 |
| 0.56-0.44 | 1.9 | 4.2 |
| 0.44-0.32 | 4.3 | 18.3 |
| 0.32-0.20 | 4.9 | 27.4 |
| 0.20-0.08 | 37.6 | 15.4 |
| 0.08-0.00 | 50.9 | 9.0 |

As is obvious from the foregoing, the amino acid-containing fat emulsion according to this invention is superior to any conventional product particularly in storage stability.

Claims:

1.      An amino acid-containing fat emulsion comprising at least one member selected from a group consisting of N-acyl-lysine, citrulline, N-acyl-arginine and N-acyl-ornithine.

2.      The fat emulsion of claim 1, comprising at least one member of essential amino acids or derivatives thereof.

3.      The fat emulsion of claim 1, comprising all members of essential amino acids or derivatives thereof.

4.      The fat emulsion of claim 1, wherein said fat emulsion comprises threonine, serine, proline, glycine, alanine, valine, methionine, isoleucine, leucine, tryptophan, phenylalanine, histidine, lysine or derivatives thereof, citrulline and cysteine.

5.      The fat emulsion of claim 1, having an amino acid content of 1 to 15 W/V %.

6.      The fat emulsion of claim 1, having a fat content of 1 to 15 W/V %.

7.      The fat emulsion of claim 1, wherein N-acyl-lysine is present in the concentration of $4 \times 10^{-3}$ to $5 \times 10^{-4}$ % (mole/V).

8.      A fat emulsion of claim 1, wherein citrulline, N-acyl-arginine or N-acyl-ornithine is present in the concentration of $4 \times 10^{-3}$ to $5 \times 10^{-4}$ % (mole/V).

9.      The fat emulsion of claim 1, further containing a carbohydrate.

- 17 -

10.    The fat emulsion of claim 9, wherein said carbohydrate contains at least one member selected from a group consisting of sorbitol, xylitol and glycerin.

0144434

# INTERNATIONAL SEARCH REPORT

International Application No. PCT/JP84/00196

## I. CLASSIFICATION OF SUBJECT MATTER (if several classification symbols apply, indicate all) [3]

According to International Patent Classification (IPC) or to both National Classification and IPC

Int. Cl[3] A61K 9/10, A61K 31/195, A61K 31/23

## II. FIELDS SEARCHED

### Minimum Documentation Searched [4]

| Classification System | Classification Symbols |
|---|---|
| IPC | A61K 9/10, A61K 31/195, A61K 31/23 |

### Documentation Searched other than Minimum Documentation to the Extent that such Documents are included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| A | JP, A, 55-38314 (Tanabe Seiyaku Co., Ltd.) 20 February 1982 (20. 02. 82) | 1 - 10 |
| P | JP, A, 59-59658 (Johnson & Johnson Products Inc.) 5 April 1984 (05. 04. 84) & PT, A, 77227 | 1 - 10 |
| A | America Ishikai-hen, Iyakuhin no Hyoka, Tekisei Shiyou no Jissai, Hirokawa Shoten, 30 November 1979 (30. 11. 79) P251-252, Vivonex Standard Diet (Eaten) no ko oyobi Vivonex HN (Eaten) no ko & AMA Drug Evaluations, Third Edition Sansho | 1 - 10 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

Date of the Actual Completion of the International Search [2]

July 17, 1984 (17. 07. 84)

Date of Mailing of this International Search Report [2]

July 30, 1984 (30. 07. 84)

International Searching Authority [1]

Japanese Patent Office

Signature of Authorized Officer [20]

Form PCT/ISA/210 (second sheet) (October 1981)